# EUROPEAN PATENT SPECIFICATION

(11) **EP 3 777 818 B1**
(45) Date of publication and mention of the grant of the patent: **02.12.2026**
(21) Application number: 20190597.3
(22) Date of filing: 11.08.2020
(51) Int. Cl.: A61K 8/02, A61K 8/81, A61K 8/891, A61K 8/88, A61Q 17/04, A61Q 1/02

(54) **DISPERSIBLE POWDER AND COSMETIC**
DISPERGIERBARES PULVER UND KOSMETIKUM
POUDRE DISPERSIBLE ET COMPOSITION COSMÉTIQUE

(30) Priority: 16.08.2019 JP 2019149395
(43) Date of publication of application: 17.02.2021
(73) Proprietor: Shin-Etsu Chemical Co., Ltd., Tokyo 100-0005 (JP)
(72) Inventor: Moriya, Hiroyuki, Gunma (JP); Hayakawa, Chihiro, Tokyo (JP); Tanaka, Mitsuru, Tokyo (JP)
(74) Representative: Wibbelmann, Jobst

(56) References cited:
- EP-A1- 1 914 264
- JP-A- 2004 137 199
- JP-A- 2004 189 652
- JP-A- 2005 194 212
- JP-A- 2013 139 400
- US-A1- 2015 272 838

## Description

### TECHNICAL FIELD

The present invention relates to a cosmetic comprising a dispersible powder and water as defined in the claims.

### BACKGROUND ART

Conventionally, studies have been conducted to improve the dispersibility of powders and for the application when powders are blended into compositions.

For example, powder-surface treatments have been examined to improve the dispersibility into aqueous media and oil materials. Patent Document 1 states that treating silsesquioxane particles with silicone improves the dispersibility into silicone oil, but the dispersibility into aqueous medium is not described. Moreover, the effect and usefulness of blending the resulting particles into a composition, particularly cosmetic composition, are not described, either.

Meanwhile, Patent Document 2 describes a method in which pigment surface is hydrophilized with a silane compound having a polyoxyalkylene group. However, this method makes the surface hydrophilicity too high, and brings about problems that the powder agglomerates, and that the tactile feel upon the application of a cosmetic blended therewith is degraded.

Further, Patent Documents 3, 4 each describe an aqueous make-up cosmetic containing a high-HLB surfactant and a powder. These are mixed in a cosmetic composition to prepare a cosmetic. However, this method cannot suppress the agglomeration of the powder particles, and there are problems that spreadability upon application and stretchability are degraded, for example. Furthermore, Patent Document 5 discloses a powder having a phosphorylcholine group introduced to the powder surface. However, although this powder is dispersible in water, there are problems, for example, that the surface hydrophilicity is so high that the powder agglomerates and the stability is degraded over time, and that the powder absorbs water in air, causing the user to feel heavy when using the powder.
Patent Document 6 discloses an organic-inorganic hybrid powder comprising an inorganic powder coated with a biocompatible polymer.
Patent Document 7 discloses a modified powder coated with a specific copolymer represented by formula 1. Patent Document 8 discloses a composite functional powder having a light transmitting property and wherein a diffuse reflecting property is improved by coating a light-transmitting organic polymer powder having a specific shape with a plate-like inorganic powder and a water-soluble polymer.
Patent Document 9 discloses a powder coating agent comprising a 2-methacryloyloxyethyl phosphorylcholine copolymer represented by formula (1) and having a weight average molecular weight of 5,000 to 1,000,000.
Patent Document 10 discloses a surface-treated powder prepared by coating the surface of a powder comprising a silicone resin and/or a specific organic powder with a specific hydrophilizing agent.

### CITATION LIST

### PATENT LITERATURE

Patent Document 1: Japanese Unexamined Patent Application Publication No. Hei 1-266141
Patent Document 2: Japanese Unexamined Patent Application Publication No. 2003-26958
Patent Document 3: Japanese Unexamined Patent Application Publication No. 2001-220319
Patent Document 4: Japanese Unexamined Patent Application Publication No. 2001-114649
Patent Document 5: Japanese Unexamined Patent Application Publication No. 2006-8661
Patent Document 6: US 2015/272838 A1
Patent Document 7: JP 2004-189652 A
Patent Document 8: JP 2005-194212 A
Patent Document 9: JP 2004-137199 A
Patent Document 10: EP 1 914 264 A1

### SUMMARY OF INVENTION

### TECHNICAL PROBLEM

The present invention has been made in view of the above-described problems. An object of the present invention is to provide a cosmetic comprising a dispersible powder having excellent dispersibility and high dispersion stability particularly in an aqueous medium, the cosmetic blended with this powder being capable of forming a uniform cosmetic film and having favorable spreadability upon application, stretchability, adhesiveness, and feeling on use as well as high stability.

### SOLUTION TO PROBLEM

To achieve the object, the present invention provides a cosmetic as defined in the claims comprising water and a dispersible powder comprising a powder treated with a surface treatment agent as defined in the claims.

The dispersible powder used in the cosmetic according to the invention has excellent dispersibility and high dispersion stability particularly in an aqueous medium. Moreover, the inventive dispersible powder exhibits high dispersibility even when dispersed in a non-aqueous cosmetic.

The surface treatment agent comprises a nonionic surfactant as defined in the claims.

The use of such a surface treatment agent makes the powder more excellent in dispersibility.

The nonionic surfactant has an HLB of 8 to 18 and is preferably selected from sorbitan fatty acid ester, glycerin fatty acid ester, polyglycerin fatty acid ester, propylene glycol fatty acid ester, polyethylene glycol fatty acid ester, sucrose fatty acid ester, methyl glucoside fatty acid ester, alkyl polyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesterol ether, polyether-modified organopolysiloxane, and polyglycerin-modified organopolysiloxane.

Moreover, the nonionic surfactant is preferably polyglycerin fatty acid ester.

In this case, the polyglycerin fatty acid ester is preferably polyglyceryl isostearate.

In the present invention, such a nonionic surfactant is suitably utilizable.

Further, in this case, the polyglyceryl isostearate has an HLB of 8 to 18.

Such polyglyceryl isostearate can further improve the dispersibility of the dispersible powder in an aqueous medium.

Furthermore, the powder is at least one silicone powder selected from (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane, and polyarylsilsesquioxane

In the present invention, such powders are suitably usable.

The present invention thus provides a cosmetic as defined in the claims comprising the above-described dispersible powder and water.

Such a cosmetic is a cosmetic excellent in dispersibility, high in dispersion stability, capable of forming a uniform cosmetic film, and having favorable spreadability upon application, stretchability, adhesiveness, and feeling on use.

The cosmetic comprises water.

Thereby, the cosmetic can have favorable feeling on use and excellent usability and persistency.

Additionally, the cosmetic preferably further comprises a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol.

This provides the cosmetic with more favorable feeling on use and excellent usability and persistency.

### ADVANTAGEOUS EFFECTS OF INVENTION

As described above, the inventive cosmetic comprising the dispersible powder is excellent in dispersibility, high in dispersion stability, capable of forming a uniform cosmetic film when blended in a cosmetic, and has favorable spreadability upon application, stretchability, adhesiveness, and feeling on use.

### DESCRIPTION OF EMBODIMENTS

As stated above, there have been demands for a cosmetic comprising a dispersive powder treated to be excellent in dispersibility, high in dispersion stability, and capable of forming a uniform cosmetic film when blended in a cosmetic, thereby the cosmetic having favorable spreadability upon application, stretchability, adhesiveness, and feeling on use.

The present inventors have earnestly studied to achieve the above-described objects and consequently found that a powder surface-treated with a (meth)acrylic polymer containing a repeating unit having a phosphorylcholine group, and a nonionic surfactant having an HLB of 8 to 18 in addition to the (meth) acrylic polymer have excellent dispersion stability. In addition, no such dispersible powders treated with the surface treatment agent(s) have been added to cosmetics.

Further, the inventors have found that an inventive cosmetic blended with the dispersible powder has favorable spreadability upon application and favorable stretchability, adhesiveness, and feeling on use. These findings have led to the present invention.

Specifically, the present invention is a cosmetic comprising a dispersible powder and water as defined in the claims.

Hereinafter, the present invention will be described in detail.

### [Dispersible Powder]

The dispersible powder used in the cosmetic is a dispersible powder including a powder treated with a surface treatment agent as defined in the claims. The surface treatment agent contains a (meth)acrylic polymer having a repeating unit with a phosphorylcholine group. Hereinbelow, the dispersible powder will be described.

### [Powder]

The powder used in the present invention is at least one silicone powder selected from (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane, and polyarylsilsesquioxane.

Examples of the silicone powder include (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, (PEG-15/lauryl dimethicone) crosspolymer, polyalkylsilsesquioxane (for example, polymethylsilsesquioxane), and polyarylsilsesquioxane.

The silicone powder is more preferably (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane, or polyarylsilsesquioxane.

The particle shape of the powder may be spherical irregular, concaved, or cup-like shape. The powder has an average particle diameter of preferably 0.5 to 100 µm, more preferably 1 to 50 µm. Note that, unless otherwise particularly specified, the average particle diameter according to the present invention refers to a volume average particle diameter that can be measured through observation with an optical microscope or a scanning electron microscope. With the average particle diameter in the above-described ranges, a cosmetic containing the inventive dispersible powder has more favorable stretchability, and a cosmetic film formed by applying and spreading the cosmetic has more favorable uniformity.

### [Surface Treatment Agent]

The surface treatment agent used in the present invention contains a (meth)acrylic polymer containing a repeating unit having a phosphorylcholine group. Herein, the polymer includes a copolymer. Hereinafter, the term polymer is also referred to as (co)polymer.

An example of a monomer for obtaining the repeating unit having a phosphorylcholine group includes one shown by the following general formula (1). 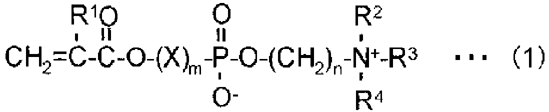 In the formula (1), X represents an alkyleneoxy group having 2 to 4 carbon atoms. R¹ represents a hydrogen atom or a methyl group. R², R³, and R⁴ represent identical or different groups among a hydrogen atom, hydrocarbon groups having 1 to 3 carbon atoms, and hydroxyhydrocarbon groups having 1 to 3 carbon atoms. "m" represents 0 or 1, and "n" represents an integer of 2 to 4.

In the formula (1), examples of X include a methyleneoxy group, an ethyleneoxy group, a propyleneoxy group, an isopropyleneoxy group, a butyleneoxy group, and a 2-butyleneoxy group; preferably an ethyleneoxy group. R¹ represents a hydrogen atom or a methyl group. Examples of R², R³, and R⁴ include a methyl group, an ethyl group, a propyl group, an isopropyl group, a hydroxymethyl group, a hydroxyethyl group, a hydroxypropyl group, and a 2-hydroxypropyl group; preferably a methyl group and a hydroxyethyl group.

"m" is 0 or 1, and "n" is an integer of 2 to 4, particularly preferably m=1 and n=2.

The (meth)acrylic (co)polymer containing the repeating unit having a phosphorylcholine group may contain another monomer unit component that has a radical polymerizable vinyl group. Examples of the vinyl monomer include lower alkyl (meth)acrylates such as methyl (meth)acrylate, ethyl (meth)acrylate, n-propyl (meth) acrylate, isopropyl (meth)acrylate, n-butyl (meth) acrylate, isobutyl (meth)acrylate, tert-butyl (meth) acrylate, n-hexyl (meth)acrylate, and cyclohexyl (meth)acrylate; higher alkyl (meth)acrylates such as 2-ethylhexyl (meth)acrylate, octyl (meth)acrylate, lauryl (meth) acrylate, and stearyl (meth) acrylate; fatty acid vinyl esters such as vinyl acetate, vinyl propionate, vinyl butyrate, vinyl caproate, vinyl 2-ethylhexanoate, vinyl laurate, and vinyl stearate; aromatic monomers such as styrene, vinyltoluene, benzyl (meth)acrylate, and phenoxyethyl (meth)acrylate; amide group-containing vinyl monomers such as (meth)acrylamide, N-methylol (meth)acrylamide, N-methoxymethyl (meth)acrylamide, isobutoxymethoxy (meth)acrylamide, N,N-dimethyl (meth)acrylamide, vinyl pyrrolidone, and N-vinyl acetamide; hydroxy group-containing vinyl monomers such as hydroxyethyl (meth)acrylate, hydroxypropyl (meth) acrylate, glyceryl (meth)acrylate, and hydroxyethyl acrylamide; ether bond-containing vinyl monomers such as tetrahydrofurfuryl (meth)acrylate, butoxyethyl (meth)acrylate, ethoxydiethylene glycol (meth) acrylate, polyethylene glycol (meth) acrylate, polypropylene glycol mono(meth)acrylate, hydroxybutyl vinyl ether, cetyl vinyl ether, and 2-ethylhexyl vinyl ether; glycidyl (meth)acrylate, (meth)allyl glycidyl ether, methacryloyloxyethyl isocyanate, (meth)acrylic propyl dimethylpolysiloxane, etc.

Examples of the (meth)acrylic (co)polymer containing the repeating unit obtained from the monomer shown by the general formula (1) include Lipidure series manufactured by NOF Corporation. Examples thereof include Lipidure-PMB (polyquaternium-51), Lipidure-PMB (Ph10) (polyquaternium-51), Lipidure-PMB (BG) (polyquaternium-51), Lipidure-PMB (Ph10)-1M (polyquaternium-51), Lipidure-B (polyquaternium-51), Lipidure-HM (polymethacryloyloxyethyl phosphorylcholine), Lipidure-HM-500 (polymethacryloyloxyethyl phosphorylcholine), Lipidure-A (polyquaternium-65), Lipidure-C (polyquaternium-64), Lipidure-S (polyquaternium-61), Lipidure-NR (polyquaternium-61), and Lipidure-NA (polyquaternium-61).

As the method for obtaining the (meth)acrylic (co)polymer containing the repeating unit obtained from the monomer shown by the general formula (1), there is a method in which polymerization is conducted in the presence of the monomer and a radical polymerization initiator such as benzoyl peroxide, lauroyl peroxide, and azobisisobutyronitrile. As the polymerization method, it is possible to employ any method including solution polymerization, emulsion polymerization, suspension polymerization, and bulk polymerization. Among these, solution polymerization is a preferable method because it makes easy to appropriately adjust the variance in a graph of gel permeation chromatography (GPC) in the determination of a weight-average molecular weight to be obtained and the weight-average molecular weight of the copolymer.

The polymerization reaction may be performed in a solvent. Examples of the solvent used in the polymerization include aliphatic organic solvents such as pentane, hexane, decane, dodecane, hexadecane, and octadecane; aromatic organic solvents such as benzene, toluene, and xylene; alcohol-based organic solvents such as methanol, ethanol, propanol, butanol, hexanol, and decanol; halogenated organic solvents such as chloroform and carbon tetrachloride; and ketone-based organic solvents such as acetone and methyl ethyl ketone. Nevertheless, from the viewpoint of cosmetic usage, the reaction is preferably performed without solvent, or with ethanol or isopropanol.

The (meth)acrylic (co)polymer containing the repeating unit obtained from the monomer shown by the general formula (1) has a weight-average molecular weight of, for example, 5,000 to 2,000,000, preferably 50,000 to 1,500,000. With the weight-average molecular weight of 5,000 or more, favorable dispersibility in a cosmetic is reliably exhibited. Meanwhile, with the weight-average molecular weight of 2,000,000 or less, neither stickiness nor heaviness would be perceived. Note that, in the present invention, the weight-average molecular weight can be determined by gel permeation chromatography (GPC) analysis as weight-average molecular weight in terms of polystyrene (hereinafter the same).

The surface treatment agent used in the present invention further contains a nonionic surfactant. The nonionic surfactant has a polyoxyethylene group or a hydroxy group as a hydrophilic group. Examples of the nonionic surfactant include sorbitan fatty acid ester, glycerin fatty acid ester, polyglycerin fatty acid ester, propylene glycol fatty acid ester, polyethylene glycol fatty acid ester, sucrose fatty acid ester, methyl glucoside fatty acid ester, alkyl polyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester (for example, PEG-20 sorbitan cocoate, PEG-20 sorbitan isostearate), polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene phytostanol ether, polyoxyethylene phytosterol ether (for example, PEG-5 soy sterol), polyoxyethylene cholestanol ether, polyoxyethylene cholesterol ether, polyether-modified organopolysiloxane, and polyglycerin-modified organopolysiloxane.

The nonionic surfactant is preferably sorbitan fatty acid ester, polyglycerin fatty acid ester, polyethylene glycol fatty acid ester, sucrose fatty acid ester, methyl glucoside fatty acid ester, alkyl polyglucoside, polyoxyethylene alkyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene phytostanol ether, polyoxyethylene phytosterol ether (for example, PEG-5 soy sterol), polyoxyethylene cholestanol ether, polyoxyethylene cholesterol ether, polyether-modified organopolysiloxane, or polyglycerin-modified organopolysiloxane. The polyether-modified organopolysiloxane and polyglycerin-modified organopolysiloxane may have a branched siloxane structure.

The nonionic surfactant is more preferably polyglycerin fatty acid ester. Examples thereof include polyglyceryl-6 isostearate, polyglyceryl-10 isostearate, polyglyceryl-10 diisostearate, polyglyceryl-6 laurate, and PEG-5 glyceryl stearate. In the present invention, the nonionic surfactant has an HLB of preferably 8 to 18, more preferably 8 to 16. With the HLB of 8 or more, the affinity between the dispersible powder and an aqueous medium is not low, so that the dispersion is facilitated. Meanwhile, with the HLB of 18 or less, the surfactant will not be separated from the dispersible powder considerably, so that the dispersion of the dispersible powder into an aqueous medium is facilitated. Note that the HLB value in the present invention can be a value calculated according to the Griffin equation, for example.

### [Method for Producing Dispersible Powder]

The method for producing the dispersible powder is not particularly limited, and may be carried out according to publicly known and used equipment and treatment method. An example of the method includes a method conducting drying process, wetting process, or the like in which a powder is coated with the (meth)acrylic (co)polymer containing a repeating unit having a phosphorylcholine group and a nonionic surfactant. As the equipment, a bead mill, a hammer mill, a Nauta mixer, or the like can be used. Heating can also be performed during the surface treatment, and the temperature is preferably 30 to 150°C, particularly preferably 30 to 110°C. Note that, during the treatment with the surface treatment agent, an optional component such as water and an alcohol can be added as necessary. To obtain a desired dispersible powder, operations such as stirring, pulverization, and drying can also be performed as necessary.

In the powder surface treatment, the (meth)acrylic (co)polymer containing a repeating unit having a phosphorylcholine group can be added in an amount of 0.01 to 20 parts by mass, preferably 0.05 to 10 parts by mass, further preferably 0.1 to 5 parts by mass, based on 100 parts by mass of the powder. When the added amount is within these ranges, the dispersibility and dispersion stability are favorable.

In the powder surface treatment, the nonionic surfactant can be added in an amount of 0.1 to 40 parts by mass, preferably 0.5 to 20 parts by mass, further preferably 1.0 to 10 parts by mass, based on 100 parts by mass of the powder. When the added amount is within these ranges, the dispersibility and dispersion stability become more favorable.

### [Cosmetic]

The present invention provides a cosmetic containing the above-described dispersible powder (hereinafter also referred to as "the inventive cosmetic"). The inventive cosmetic can be blended with a medium that is allowable for cosmetics.

The content of the above-described dispersible powder is preferably 0.1 to 90 mass%, more preferably 0.2 to 50 mass%, further preferably 0.5 to 20 mass%, relative to the whole cosmetic. When the content of the dispersible powder is within these ranges, the resulting cosmetic can be felt more soft and stretchable without powdery feel, and has high stability.

The inventive cosmetic further contains water, besides the dispersible powder. This enables the inventive cosmetic to have favorable feeling on use and excellent usability and persistency.

The water content is preferably 1 to 98 mass%, more preferably 2 to 90 mass%, further preferably 3 to 80 mass%, of the whole cosmetic. When the water content is within the ranges, a cosmetic with more favorable stability and usability is obtained.

More preferably, the inventive cosmetic further contains, besides the dispersible powder and water, a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol. Thereby, the inventive cosmetic has more favorable feeling on use and excellent usability and persistency.

These hydrophilic substances are each contained in an amount of preferably 1 to 80 mass%, more preferably 1 to 40 mass%, further preferably 3 to 30 mass%, relative to the whole cosmetic. When the content of the hydrophilic substance is within these ranges, a cosmetic with more favorable stability and usability is obtained.

The inventive cosmetic may contain a volatile silicone oil or a volatile organic oil. Examples of the volatile silicone oil include dimethylpolysiloxanes (dimer, trimer, tetramer, pentamer), caprylyl methicone, octamethylcyclotetrasiloxane, decamethylcyclopentasiloxane, dodecamethylcyclohexasiloxane, tetramethyltetrahydrogencyclotetrasiloxane, tristrimethylsiloxymethylsilane, tetrakistrimethylsiloxysilane, etc.

The volatile organic oil may be a hydrocarbon oil or ester. Examples thereof include α-olefin oligomer, light isoparaffin, isododecane, light liquid isoparaffin, butyl acetate, etc. Preferable examples are dimethylpolysiloxanes (tetramer, pentamer), caprylyl methicone, tristrimethylsiloxymethylsilane, tetrakistrimethylsiloxysilane, light isoparaffin, isododecane, light liquid isoparaffin, and butyl acetate. These volatile oils are each contained in an amount of preferably 1 to 98 mass%, more preferably 1 to 50 mass%, further preferably 3 to 30 mass%, relative to the whole cosmetic.

The inventive cosmetic can further contain an oil material that is allowable for cosmetics, in addition to the volatile oil. Examples of such a liquid oil material include one or more kinds of silicone oil, hydrocarbon oil, higher fatty acid, higher alcohol, polar oils such as ester oil, glyceride oil, and natural animal and vegetable oils, semisynthetic oil, and/or fluorinated oil, etc.

Examples of the silicone oil include linear or branched organopolysiloxanes having low viscosity to high viscosity such as nonvolatile dimethylpolysiloxane, phenyltrimethicone, methylphenylpolysiloxane, dimethylsiloxy phenyl trimethicone, diphenylsiloxy phenyl trimethicone (for example, KF-56A manufactured by Shin-Etsu Chemical Co., Ltd.), methylhexylpolysiloxane, methylhydrogenpolysiloxane, and dimethylsiloxane/methylphenylsiloxane copolymer; tetramethyltetraphenylcyclotetrasiloxane, amino-modified organopolysiloxane; silicone rubbers such as highly polymerized gummy dimethylpolysiloxane, gummy amino-modified organopolysiloxane, and gummy dimethylsiloxane/methylphenylsiloxane copolymer; a solution of silicone gum or rubber in cyclic siloxane; trimethylsiloxy silicic acid, and a solution of trimethylsiloxy silicic acid in cyclic organopolysiloxane; higher alkoxy-modified organopolysiloxanes such as stearoxy silicone; higher fatty acid-modified organopolysiloxane, alkyl-modified organopolysiloxane, long chain alkyl-modified organopolysiloxane, fluorine-modified organopolysiloxane, silicone resin, silicone resin solutions, etc.

Examples of the hydrocarbon oil include nonvolatile ones such as ozocerite, squalane, synthetic squalane, vegetable squalane, squalene, ceresin, paraffin, paraffin wax, polyethylene wax, polyethylene/polypropylene wax, (ethylene/propylene/styrene) copolymer, (butylene/propylene/styrene)copolymer, liquid paraffin, liquid isoparaffin, pristane, polyisobutylene, hydrogenated polyisobutene, microcrystalline wax, vaseline, etc.

Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, etc. Examples of the higher alcohol include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, POE cholesterol ether, monostearyl glycerin ether (batyl alcohol), monooleyl glyceryl ether (selachyl alcohol), etc.

Examples of the ester oil include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyl dodecyl gum ester, oleyl oleate, octyldodecyl oleate, decyl oleate, neopentyl glycol dioctanoate, neopentyl glycol dicaprate, coco-(caprate/caprylate), triethyl citrate, 2-ethylhexyl succinate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate ester, isopropyl lauroylsarcosinate ester, diisostearyl malate, etc. Examples of the glyceride oil include acetoglyceryl, glyceryl triisooctanoate, glyceryl triisostearate, glyceryl triisopalmitate, glyceryl monostearate, glyceryl di-2-heptylundecanoate, glyceryl trimyristate, diglyceryl myristate isostearate, glyceryl triethylhexanoate, etc.

Examples of the natural animal and vegetable oils and semisynthetic oils include avocado oil, linseed oil, almond oil, Chinese wax, perilla oil, olive oil, cacao butter, kapok wax, kaya oil, carnauba wax, cod-liver oil, candelilla wax, purified candelilla wax, beef tallow, neats-foot oil, beef bone fat, cured beef tallow, apricot kernel oil, whale wax, hydrogenated oil, wheat germ oil, sesame oil, rice germ oil, rice bran oil, sugarcane wax, sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, pig fat, rapeseed oil, Japanese tung oil, bran wax, germ oil, horse fat, persic oil, palm oil, palm kernel oil, castor oil, cured castor oil, methyl ester of castor oil fatty acid, sunflower oil, grape seed oil, bayberry wax, jojoba oil, hydrogenated jojoba oil, macademia nut oil, bees wax, mink oil, meadowfoam seed oil, cotton seed oil, cotton wax, Japan wax, Japan wax kernel oil, montan wax, coconut oil, cured coconut oil, tri-coconut fatty acid glyceride, mutton tallow, peanut oil, lanolin, liquid lanolin, reduced lanolin, lanolin alcohol, hard lanolin, lanolin acetate, lanolin alcohol acetate, isopropyl lanolin fatty acid, egg-yolk oil, etc.

Examples of the fluorinated oil material include perfluoro polyether, perfluoro decalin, perfluoro octane, etc.

In the present invention, the oil material that is allowable for cosmetics, other than the volatile oil, is blended in an amount of suitably 1 to 98 mass%, preferably 1 to 50 mass%, further preferably 3 to 30 mass%, relative to the whole cosmetic, although the amount varies depending on the form of the cosmetic.

The inventive cosmetic can further contain one or more kinds of ultraviolet (UV) absorber and/or UV-scattering agent. This makes the inventive cosmetic not only have favorable feeling on use and excellent usability and persistency, but also capable of blocking ultraviolet ray.

Examples of the UV-absorber include benzoic acid UV-absorbers such as para-amino benzoic acid; anthranilic acid UV-absorbers such as methyl anthranilate; salicylic acid UV-absorbers such as methyl salicylate; cinnamic acid UV-absorbers such as ethylhexyl para-methoxycinnamate; benzophenone UV-absorbers such as 2,4-dihydroxybenzophenone; urocanic acid UV-absorbers such as ethyl urocanate; triazine UV-absorber such as bis-ethylhexyloxyphenol methoxyphenyl triazine; dibenzoylmethane UV-absorbers such as 4-t-butyl-4'-methoxy-dibenzoylmethane; etc. It is also possible to use silicone derivatives having an UV-absorbing functional group described above.

Examples of the UV-scattering agent include particles that absorb and scatter ultraviolet light such as titanium oxide microparticles, iron-containing titanium oxide microparticles, zinc oxide microparticles, cerium oxide microparticles, and composites thereof.

Among them, preferable are cinnamic acid UV-absorbers, dibenzoylmethane UV-absorbers, titanium oxide microparticles (for example, SPD-T5 manufactured by Shin-Etsu Chemical Co., Ltd.), and zinc oxide microparticles (for example, SPD-Z5 manufactured by Shin-Etsu Chemical Co., Ltd.; etc.).

The inventive cosmetic can further contain one or more kinds of surfactant that is distinguished from the above-described nonionic surfactant serving as the surface treatment agent. Thereby, the inventive cosmetic is allowed to be more excellent in emulsification stability and usability depending on the object. The surfactant includes anionic, cationic, nonionic, and amphoteric surfactants. The inventive cosmetic can employ, without particular limitation, any surfactant that is used for ordinal cosmetics.

Specific examples of the anionic surfactant include fatty acid soap such as sodium stearate and triethanolamine palmitate; alkyl ether carboxylic acid and a salt thereof, a condensation salt of amino acid and fatty acid, an alkane sulfonate salt, an alkene sulfonate salt, a sulfonate salt of fatty acid ester, a sulfonate salt of fatty acid amide, a sulfonate salt of formalin condensate; an alkyl sulfate ester salt, a sulfate ester salt of secondary higher alcohol, a sulfate ester salt of alkyl and allyl ether, a sulfate ester salt of fatty acid ester, a sulfate ester salt of fatty acid alkylolamide, a sulfate ester salt of Turkey red oil; an alkyl phosphate salt, an ether phosphate salt, an alkyl ally ether phosphate salt, an amide phosphate salt, an N-acyl lactate salt, an N-acylsarcosinate salt, an N-acylamino acid activator, carboxy vinyl polymer, polyacryl amide-based anionic surfactants, etc. Examples of the cationic surfactant include an alkyl amine salt, an amine salt of polyamine, an amino alcohol fatty acid derivative and the like, an alkyl quaternary ammonium salt (for example, behentrimonium chloride), an aromatic quaternary ammonium salt, a pyridinium salt, an imidazolium salt, etc.

Examples of the nonionic surfactant include sorbitan fatty acid ester, glycerin fatty acid ester, polyglycerin fatty acid ester, propylene glycol fatty acid ester, polyethylene glycol fatty acid ester, sucrose fatty acid ester, methyl glucoside fatty acid ester, alkyl polyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene phytostanol ether, polyoxyethylene phytosterol ether (for example, PEG-5 soy sterol), polyoxyethylene cholestanol ether, and polyoxyethylene cholesterol ether; linear or branched polyoxyalkylene-modified organopolysiloxane (for example, KF-6017, KF-6017P, KF-6028 manufactured by Shin-Etsu Chemical Co., Ltd.), linear or branched organopolysiloxane co-modified with polyoxyalkylene and alkyl (for example, KF-6038, KF-6048 manufactured by Shin-Etsu Chemical Co., Ltd.; etc.), linear or branched polyglycerin-modified organopolysiloxane (for example, KF-6100, KF-6104, KF-6106 manufactured by Shin-Etsu Chemical Co., Ltd.; etc.), linear or branched organopolysiloxane co-modified with polyglycerin and alkyl (for example, KF-6105 manufactured by Shin-Etsu Chemical Co., Ltd.; etc.), alkanol amide, sugar ether, sugar amide, etc.

Examples of the amphoteric surfactant include betaine, an aminocarboxylic acid salt, an imidazoline derivative, an amide amine type, etc.

The inventive cosmetic may further contain a composition composed of liquid oil material and one or more kinds of crosslinked organopolysiloxane polymer without having a hydrophilic group. The crosslinked organopolysiloxane polymer can be obtained by reaction of alkylhydrogenpolysiloxane and/or arylhydrogenpolysiloxane with a crosslinking agent that has a reactive vinyl type unsaturated group(s) at the terminal of the molecular chain.

Examples of the alkylhydrogenpolysiloxane include linear or partially branched methylhydrogenpolysiloxane, methylhydrogenpolysiloxane in which an alkyl chain with 6 to 20 carbon atoms is grafted, etc. Each of these molecules has to contain two or more hydrogen atoms that are bonded to silicon atoms in average. Examples of the crosslinking agent include a molecule having two or more vinyl type reaction moieties, such as methylvinylpolysiloxane and α,ω-alkenyldiene.

Examples thereof include compositions described in JP 1925781B, JP 1932769B, WO 03/24413A1, and JP 2009-185296A. The crosslinked organopolysiloxane polymer (for example, crosslinked methylpolysiloxane) is swollen with low viscosity silicone with the viscosity of 0.65 to 100.0 mm²/second (at 25°C), hydrocarbon oil such as liquid paraffin, squalane, and isododecane, glyceride oil such as trioctanoin, as well as ester oil in an amount of the own weight or more. Examples of the commercially products of these crosslinked organopolysiloxane include KSG-15, KSG-16, KSG-18, KSG-18A, KSG-1610, and USG-103, which are pastes mixed with silicone oil; USG-106, KSG-41, KSG-42, KSG-43, KSG-44, and KSG-810, which are pastes mixed with hydrocarbon oil or triglyceride oil (all of which are manufactured by Shin-Etsu Chemical Co., Ltd.); etc., but are not particularly limited thereto.

The composition composed of liquid oil material and crosslinked organopolysiloxane without having a hydrophilic group(s) is blended in an amount of preferably 0.1 to 50 mass%, more preferably 1 to 30 mass%, relative to the whole amount of the cosmetic.

The inventive cosmetic may further contain a composition composed of liquid oil material and one or more kinds of crosslinked organopolysiloxane polymer having a hydrophilic group. The hydrophilic group is preferably a polyether group and a polyglycerin group. The crosslinked organopolysiloxane polymer having a polyether group and/or a polyglycerin group can be obtained by reaction of alkylhydrogenpolysiloxane and a crosslinking agent that has a reactive vinyl type unsaturated group(s) at the terminal of the molecular chain. Examples of the alkylhydrogenpolysiloxane include methylhydrogenpolysiloxane in which a polyoxyethylene chain is grafted, methylhydrogenpolysiloxane in which a polyglycerin chain is grafted, etc. Each of these molecules has to contain two or more hydrogen atoms that are bonded to silicon atoms in average.

This crosslinked organopolysiloxane polymer is swollen with low viscosity silicone with the viscosity of 0.65 to 100.0 mm²/second (at 25°C), hydrocarbon oil such as liquid paraffin, squalane, and isododecane, glyceride oil such as trioctanoin, as well as ester oil in an amount of the own weight or more. Examples of the crosslinking agent include a molecule having two or more vinyl type reaction moieties such as methylvinylpolysiloxane, α,ω-alkenyldiene, glycerin triallyl ether, polyoxyalkynylated glycerin triallyl ether, trimethylolpropane triallyl ether, and polyoxyalkynylated trimethylolpropane triallyl ether, provided that the crosslinked product by the reaction therewith contains at least one hydrophilic group.

Preferable examples of the composition include ones described in JP 2631772B, JP H09-136813A1, JP 2001-342255A1, WO 03/20828A1, and JP 2009-185296A. Examples of the commercially products of these crosslinked organopolysiloxanes include KSG-210, KSG-240, and KSG-710, which are pastes mixed with silicone oil; KSG-310, KSG-320, KSG-330, KSG-340, KSG-820, KSG-830, KSG-840, and KSG-850Z which are pastes mixed with hydrocarbon oil or triglyceride oil (all of which are manufactured by Shin-Etsu Chemical Co., Ltd.); etc., but are not particularly limited thereto.

The composition composed of liquid oil material and crosslinked organopolysiloxane having a hydrophilic group(s) is blended in an amount of preferably 0.1 to 50 mass%, more preferably 1 to 30 mass%, relative to the whole amount of the cosmetic.

The inventive cosmetic may contain silicone wax in accordance with the object. This silicone wax is preferably polylactone-modified polysiloxane having bonded polylactone, which is a ring opening polymerization product of a lactone compound having a ring of five or more atoms. Alternatively, this silicone wax is preferably acrylic-modified polysiloxane with the molecule containing at least one functional group selected from a pyrrolidone group, a long-chain alkyl group, a polyoxyalkylene group, a fluoroalkyl group, and anionic groups such as a carboxylic acid. Examples of commercial products include KP-561P and KP-562P (all of which are manufactured by Shin-Etsu Chemical Co., Ltd.) as wax having a long-chain alkyl group.

The inventive cosmetic may further contain a component(s) used for ordinary cosmetics, an oil-soluble gelation agent (organic-modified clay mineral), various powders (inorganic powder, color pigment, pearl pigment, organic powder), antiperspirant, moisturizer, antimicrobial preservative, fragrance, salt, antioxidant, pH adjuster, chelating agent, cooling agent, anti-inflammatory agent, skin care component (such as whitening agent, cell activator, rough skin improver, blood circulation promoter, skin astringent, antiseborrheic agent), vitamin, amino acid, nucleic acid, hormone, inclusion compound, etc.

Examples of the oil-soluble gelation agent include one or more kinds of oil-soluble gelation agents selected from metal soap such as aluminum stearate, magnesium stearate, and zinc myristate; amino acid derivative such as N-lauroyl-L-glutamic acid and α,γ-din-butyl amine; dextrin fatty acid ester such as dextrin palmitate ester, dextrin stearate ester, and dextrin 2-ethylhexanoate palmitate ester; sucrose fatty acid ester such as sucrose palmitate ester and sucrose stearate ester; fructo-oligosaccharide fatty acid ester such as fructo-oligosaccharide stearate ester and fructo-oligosaccharide 2-ethylhexanoate ester; benzylidene derivative of sorbitol such as monobenzylidene sorbitol and dibenzylidene sorbitol; organic-modified clay mineral such as dimethyl benzyl dodecyl ammonium montomorillonite clay and dimethyl dioctadecyl ammonium hectorite; etc.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, anhydrous silicic acid, aluminum silicate, magnesium silicate, aluminum magnesium silicate, calcium silicate, barium silicate, strontium silicate, metal tungstate salt, hydroxy apatite, vermiculite, higilite, bentonite, montomorillonite, hectorite, zeolite, ceramic powder, dibasic calcium phosphate, alumina, aluminum hydroxide, boron nitride, boron nitride, silica, and silica silylate.

Examples of the color pigment include inorganic red pigment such as iron oxide, iron hydroxide, and iron titanate; inorganic brown pigment such as γ-iron oxide; inorganic yellow pigment such as yellow iron oxide and loess; inorganic black pigment such as black iron oxide and carbon black; inorganic purple pigment such as manganese violet and cobalt violet; inorganic green pigment such as chromium hydroxide, chromium oxide, cobalt oxide, and cobalt titanate; inorganic blue pigment such as Prussian blue and ultramarine blue; laked tar dye; laked natural dye; synthetic resin powder pigment obtained by hybridization of these powders; etc.

Examples of the pearl pigment include muscovite coated with titanium oxide, mica coated with titanium oxide, oxychloro bismuth, oxychloro bismuth coated with titanium oxide, talc coated with titanium oxide, fish scale foil, color mica coated with titanium oxide, etc. Examples of the metal powder pigment include aluminum powder, copper powder, stainless powder, etc.

Examples of the organic powder that may be blended include silicone powder, polyamide powder, polyacrylic acid-acrylic ester powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, nylon powder, etc., but these are not used for the surface treatment as the above-described surface treatment agent.

The powders (various powders described above) that can be added to the inventive cosmetic preferably have hydrophobic surface. The term hydrophobic means that the powder does not disperse in water. If the powder surface is hydrophobic from the beginning, the powder may be used as it is. As necessary, a powder may be hydrophobized as follows and used. The use of such hydrophobic powder improves the uniformity of the cosmetic film, settlement upon application, and adhesiveness. In addition, the various powders preferably have an average particle diameter of 200 µm or less. The particles constituting the various powders are not particularly limited in regard to the form (spherical, needle-like, plate-like, etc.) or particle structure (porous, non-porous, etc.). The average particle diameter is preferably 200 µm or less, more preferably 100 µm or less, further preferably 50 µm or less. Here, the average particle diameter refers to volume average particle diameter and can be measured by laser scattering method etc.

These various powders are preferably surface-hydrophobized with a treatment agent selected from silicone, fluorine compounds, silane coupling agents, titanium coupling agents, N-acylated amino acids, and metal soaps. Particularly, since the inorganic powder, color pigment, and pearl pigment have hydrophilic surfaces, these are preferably used after subjected to the surface hydrophobic treatment with the treatment agent selected from silicone, fluorine compounds, silane coupling agents, titanium coupling agents, N-acylated amino acids, and metal soaps, which are distinguished from the surface treatment agent essentially used in the present invention.

Examples of the antiperspirant include aluminum chlorohydrate, aluminum chloride, aluminum sesquichlorohydrate, zirconyl hydroxychloride, aluminum zirconium hydroxychloride, aluminum zirconium glycine complex, etc.

Examples of the moisturizer include sorbitol, xylitol, maltitol, polyethylene glycol, polysaccharides, hydroxyethylcellulose, xanthan gum, hyaluronic acid, chondroitin sulfate, pyrrolidone carboxylate, polyoxyethylene methyl glucoside, polyoxypropylene methyl glucoside, egg-yolk lecithin, soybean lecithin, phosphatidylcholine, phosphatidylethanolamine, phosphatidylserine, phosphatidylglycerol, phosphatidylinositol, sphingophospholipid, etc.

Examples of the antimicrobial preservative include alkyl para-oxybenzoate (paraben), benzoic acid, sodium benzoate, sorbic acid, potassium sorbate, phenoxy ethanol, etc. Examples of the antimicrobial include benzoic acid, salicylic acid, carbolic acid, sorbic acid, alkyl para-oxybenzoate, p-chloro-m-cresol, hexachlorophene, benzalkonium chloride, chlorhexidine chloride, trichlorocarbanilide, photosensitizers, phenoxy ethanol, etc.

The fragrance includes natural fragrance and synthetic fragrance. Examples of the natural fragrance include vegetable fragrances obtained by separating from flower, leaf, material, peel, and the like; and animal fragrances such as musk (bisabolol) and civet. Examples of the synthetic fragrance include hydrocarbons such as monoterpene; alcohols such as aliphatic alcohols and aromatic alcohols; aldehydes such as terpene aldehyde and aromatic aldehyde; ketones such as alicyclic ketones; esters such as terpene-based esters; lactones; phenols; oxides; nitrogen-containing compounds; acetals; etc.

Examples of the salt include inorganic salts, organic acid salts, amine salts, and amino acid salts. Examples of the inorganic salts include sodium salts, potassium salts, magnesium salts, calcium salts, aluminum salts, zirconium salts, barium salts, zinc salts, and the like of inorganic acid such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and the amino acid salts include salts of amines such as triethanolamine, and salts of amino acids such as glutamic acid; etc. In addition, salts of hyaluronic acid, chondroitin sulfate, aluminum zirconium glycine complex, an acid-alkali neutral salt used in cosmetic preparation, or the like can be used.

Examples of the antioxidant include tocopherol, p-t-butylphenol, butylhydroxyanisol, dibutylhydroxytoluene (BHT), phytic acid, etc.

Examples of the pH adjuster include lactic acid, citric acid, glycolic acid, succinic acid, tartaric acid, dl-malic acid, potassium carbonate, sodium bicarbonate, ammonium bicarbonate, etc.

Examples of the chelating agent include alanine, sodium edetate, sodium polyphosphate, sodium metaphosphate, phosphoric acid, etc.

Examples of the cooling agent include L-menthol, camphor, etc.

Examples of the anti-inflammatory agent include allantoin, glycyrrhizinic acid and salt thereof, glycyrrhetinic acid and stearyl glycyrrhetinate, tranexamic acid, azulene, etc.

Examples of the skin care component (such as whitening agent, cell activator, rough skin improver, blood circulation promoter, skin astringent, antiseborrheic agent) include whitening agents such as placenta extract, arbutin, glutathione, and saxifragaceae extract; cell activators such as royal jelly, photosensitive element, cholesterol derivative, and young calf blood extract; rough skin improvers; blood circulation promoters such as nonanoic acid vanillylamide, benzyl nicotinate, β-butoxyethyl nicotinate, capsaicin, zingerone, cantharides tincture, ichthammol, caffeine, tannic acid, α-borneol, nicotinic acid tocopherol, inositol hexanicotinate, cyclandelate, cinnarizine, tolazoline, acetylcholine, verapamil, cepharanthine, and γ-oryzanol; skin astringents such as zinc oxide and tannic acid; antiseborrheic agents such as sulfur and thianthol; etc.

Examples of the vitamin include vitamin A such as vitamin A oil, retinol, retinol acetate, and retinol palmitate; vitamin B including vitamin B₂ such as riboflavin, riboflavin butyrate, and flavin adenine nucleotide, vitamin B₆ such as pyridoxine hydrochloride salt, pyridoxine dioctanoate, and pyridoxine tripalmitate, vitamin B₁₂ and its derivative, and vitamin B₁₅ and its derivative; vitamin C such as L-ascorbic acid, L-ascorbic acid dipalmitate ester, sodium L-ascorbic acid-2-sulfate, and dipotassium L-ascorbic acid phosphate diester; vitamin D such as ergocalciferol and cholecalciferol; vitamin E such as α-tocopherol, β-tocopherol, γ-tocopherol, dl-α-tocopherol acetate, dl-α-tocopherol nicotinate, and dl-α-tocopherol succinate; nicotinic acid such as nicotinic acid, benzyl nicotinate, and nicotinic acid amide; vitamin H; vitamin P; pantothenic acid such as calcium pantothenate, D-pantothenyl alcohol, pantothenyl ethyl ether, and acetyl pantothenyl ethyl ether; biotin; etc.

Examples of the amino acid include glycine, valine, leucine, isoleucine, serine, threonine, phenylalanine, arginine, lysine, aspartic acid, glutamic acid, cystine, cysteine, methionine, and tryptophan, etc.

Examples of the nucleic acid include deoxyribonucleic acid, etc.

Examples of the hormone include estradiol, ethenyl estradiol, etc.

Examples of the inclusion compound include cyclodextrin, etc.

The inventive cosmetic includes cosmetics in which the cosmetic component(s) described above are blended, such as make-up cosmetics including skin care cosmetic, milky lotion, cream, make-up foundation, concealer, white powder, liquid foundation, oil foundation, rouge, eye shadow, mascara, eye liner, eye blow, and lipstick; hair cosmetics including shampoo, rinse, treatment, and setting material; UV-protective cosmetics including antiperspirant, sunscreen oil, sunscreen lotion, and sunscreen cream; etc.

These cosmetics may be in various forms, such as liquid, milky lotion, cream, solid, paste, gel, powder, pressed, laminated, mousse, spray, and stick forms.

Further, these cosmetics may be in various types, such as a water-base, an oil-base, an oil-in-water emulsion, a water-in-oil emulsion, a non-aqueous emulsion, a multi-emulsion including W/O/W and O/W/O.

### EXAMPLE

Hereinafter, the present invention will be specifically described with reference to Examples of the dispersible powder and cosmetic, and Comparative Examples. However, the present invention is not limited to the following Examples. Incidentally, unless otherwise especially noted, "%" described below means "mass%", and mass% of each component is expressed with the total mass in each example being taken as 100%.

### <Example 1> (not claimed)

Into a flask, 100 g of polymethylsilsesquioxane (average particle diameter: 5 µm), 2 g of Lipidure-PMB (manufactured by NOF Corporation, weight-average molecular weight: 600,000), and 50 g of ion-exchanged water were added and stirred for 1 hour. Then, with stirring, the mixture was dried under reduced pressure at 100°C for 3 hours and pulverized. Thus, a white powder was obtained. This powder well dispersed in water, and hardly agglomerated over time.

### <Example 2>

Into a flask, 100 g of (vinyl dimethicone/methicone silsesquioxane) crosspolymer (average particle diameter: 5 µm), 0.4 g of Lipidure-PMB (Ph10) (manufactured by NOF Corporation, weight-average molecular weight: 1,000,000), 5 g of polyglyceryl-10 diisostearate (HLB: 9.5), and 50 g of ion-exchanged water were added and stirred for 1 hour. Then, with stirring, the mixture was dried under reduced pressure at 100°C for 3 hours and pulverized. Thus, a white powder was obtained. This powder well dispersed in water, and hardly agglomerated over time.

### <Example 3> (not claimed)

Into a flask, 100 g of (dimethicone/vinyl dimethicone) crosspolymer (average particle diameter: 10 µm), 0.1 g of Lipidure-HM (manufactured by NOF Corporation, weight-average molecular weight: 100,000), 1 g of polyglyceryl-6 laurate (HLB: 14.5), and 10 g of ethanol were added and stirred for 1 hour. Then, with stirring, the mixture was dried under reduced pressure at 100°C for 3 hours and pulverized. Thus, a white powder was obtained. This powder well dispersed in water, and hardly agglomerated over time.

### <Example 4> (not claimed)

Into a flask, 100 g of polyamide powder (average particle diameter: 20 µm), 0.2 g of Lipidure-NR (manufactured by NOF Corporation, weight-average molecular weight: 100,000), 3 g of PEG-5 glyceryl stearate (HLB: 9.5), and 5 g of ethanol were added and stirred for 1 hour. Then, with stirring, the mixture was dried under reduced pressure at 80°C for 3 hours and pulverized. Thus, a white powder was obtained. This powder well dispersed in water, and hardly agglomerated over time.

### <Example 5> (not claimed)

Into a flask, 100 g of methyl methacrylate crosspolymer (average particle diameter: 1 µm), 0.2 g of Lipidure-PMB (manufactured by NOF Corporation, weight-average molecular weight: 600,000), 8 g of PEG-20 sorbitan cocoate (HLB: 16), and 20 g of 2-propanol were added and stirred for 1 hour. Then, with stirring, the mixture was dried under reduced pressure at 80°C for 3 hours and pulverized. Thus, a white powder was obtained. This powder well dispersed in water, and hardly agglomerated over time.

### <Example 6> (not claimed)

Into a flask, 100 g of polyurethane powder (average particle diameter: 0.5 µm), 1.0 g of Lipidure-NR (manufactured by NOF Corporation, weight-average molecular weight: 100,000), 2 g of PEG-5 soy sterol (HLB: 9.5), and 50 g of methanol were added and stirred for 1 hour. Then, with stirring, the mixture was dried under reduced pressure at 70°C for 3 hours and pulverized. Thus, a white powder was obtained. This powder well dispersed in water, and hardly agglomerated over time.

### <Example 7> (not claimed)

Into a flask, 100 g of polyethylene powder (average particle diameter: 30 µm), 0.2 g of Lipidure-PMB (manufactured by NOF Corporation, weight-average molecular weight: 600,000), 5 g of polyether-modified organopolysiloxane (HLB: 8.7) shown by the following formula, and 20 g of acetone were added and stirred for 1 hour. Then, with stirring, the mixture was dried under reduced pressure at 60°C for 3 hours and pulverized. Thus, a white powder was obtained. This powder well dispersed in water, and hardly agglomerated over time.

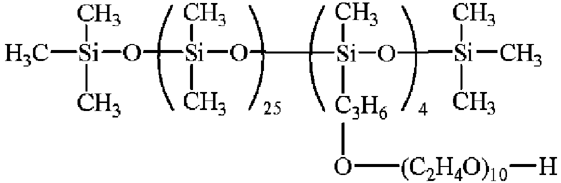

### <Example 8>

Into a flask, 100 g of (diphenyl dimethicone/vinyl diphenyl dimethicone/silsesquioxane) crosspolymer (average particle diameter: 5 µm), 2 g of Lipidure-PMB (manufactured by NOF Corporation, weight-average molecular weight: 600,000), 0.3 g of polyglycerin-modified organopolysiloxane (HLB: 8.0) shown by the following formula, and 20 g of 2-propanol were added and stirred for 1 hour. Then, with stirring, the mixture was dried under reduced pressure at 110°C for 3 hours and pulverized. Thus, a white powder was obtained. This powder well dispersed in water, and hardly agglomerated over time.

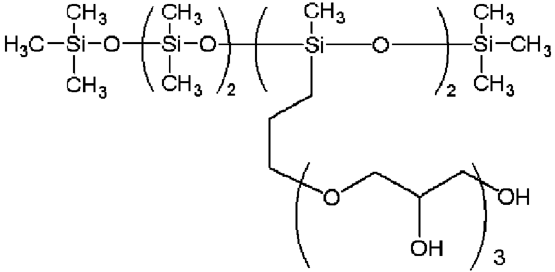

### <Example 9>

Into a flask, 100 g of (vinyl dimethicone/methicone silsesquioxane) crosspolymer (average particle diameter: 5 µm), 0.4 g of Lipidure-PMB (manufactured by NOF Corporation, weight-average molecular weight: 600,000), 5 g of polyglyceryl-10 isostearate (HLB: 10.0), and 50 g of ion-exchanged water were added and stirred for 1 hour. Then, with stirring, the mixture was dried under reduced pressure at 100°C for 3 hours and pulverized. Thus, a white powder was obtained. This powder well dispersed in water, and hardly agglomerated over time.

### <Example 10>

Into a flask, 100 g of (vinyl dimethicone/methicone silsesquioxane) crosspolymer (average particle diameter: 5 µm), 0.4 g of Lipidure-PMB (manufactured by NOF Corporation, weight-average molecular weight: 600,000), 5 g of PEG-20 sorbitan isostearate (HLB: 15.0), and 50 g of ion-exchanged water were added and stirred for 1 hour. Then, with stirring, the mixture was dried under reduced pressure at 100°C for 3 hours and pulverized. Thus, a white powder was obtained. This powder well dispersed in water, and hardly agglomerated over time.

### <Comparative Example 1>

Into a flask, 100 g of polymethylsilsesquioxane (average particle diameter: 5 µm), 2 g of polyvinyl alcohol, and 50 g of ion-exchanged water were added and stirred for 1 hour. Then, with stirring, the mixture was dried under reduced pressure at 100°C for 3 hours and pulverized. Thus, a white powder was obtained. This powder dispersed in water, but agglomerated over time.

### <Comparative Example 2>

Into a flask, 100 g of (vinyl dimethicone/methicone silsesquioxane) crosspolymer (average particle diameter: 5 µm), 5 g of polyglyceryl-10 diisostearate (HLB: 9.5), and 50 g of ion-exchanged water were added and stirred for 1 hour. Then, with stirring, the mixture was dried under reduced pressure at 100°C for 3 hours and pulverized. Thus, a white powder was obtained. This powder dispersed in water, but agglomerated over time.

### <Comparative Example 3>

Into a flask, 100 g of methyl methacrylate crosspolymer (average particle diameter: 1 µm), 8 g of PEG-20 sorbitan cocoate (HLB: 16), and 20 g of 2-propanol were added and stirred for 1 hour. Then, with stirring, the mixture was dried under reduced pressure at 80°C for 3 hours and pulverized. Thus, a white powder was obtained. This powder dispersed in water, but agglomerated over time.

### <Comparative Example 4>

Into a flask, 100 g of polyurethane powder (average particle diameter: 0.5 µm), 2 g of PEG-5 soy sterol (HLB: 9.5), and 50 g of methanol were added and stirred for 1 hour. Then, with stirring, the mixture was dried under reduced pressure at 70°C for 3 hours and pulverized. Thus, a white powder was obtained. This powder dispersed in water, but agglomerated over time.

### <Comparative Example 5>

Into a flask, 100 g of (vinyl dimethicone/methicone silsesquioxane) crosspolymer (average particle diameter: 5 µm), 5 g of polyglyceryl-10 isostearate (HLB: 10.0), and 50 g of ion-exchanged water were added and stirred for 1 hour. Then, with stirring, the mixture was dried under reduced pressure at 100°C for 3 hours and pulverized. Thus, a white powder was obtained. This powder dispersed in water, but agglomerated over time.

### <Comparative Example 6>

According to the method disclosed in JP 2006-8661A, (vinyl dimethicone/methicone silsesquioxane) crosspolymer (average particle diameter: 5 µm) was surface-treated with silane having a phosphorylcholine group shown in the following structure. Thus, a white powder was obtained. This powder dispersed in water, but agglomerated over time. Meanwhile, when left standing as it was, the powder absorbed water in air and became sticky.

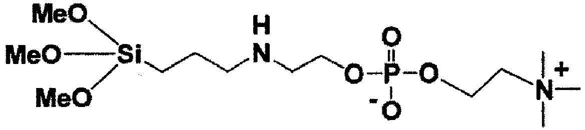

### <Examples 11, 12, Comparative Examples 7 to 9>

Dispersions shown in Table 1 were prepared to evaluate the dispersibility.

### [Evaluation Method]

According to the formulations shown in Table 1 below, the powders were tried to disperse in water or ethanol, and left alone for storage in a thermostat set under a temperature condition of 25°C, 50°C, or -5°C. Each powder sample was taken at both timings of immediately after the preparation and 1 week thereafter, and left alone in a thermostat at 25°C for 2 hours. Then, the powder was shaken together with the storage sample bottle ten times as one set. After each set of shaking was completed, whether or not the powder agglomerated was visually evaluated.

### [Evaluation Criteria]

good: dispersed after 1 or 2 sets
poor: dispersed after 3 or more sets

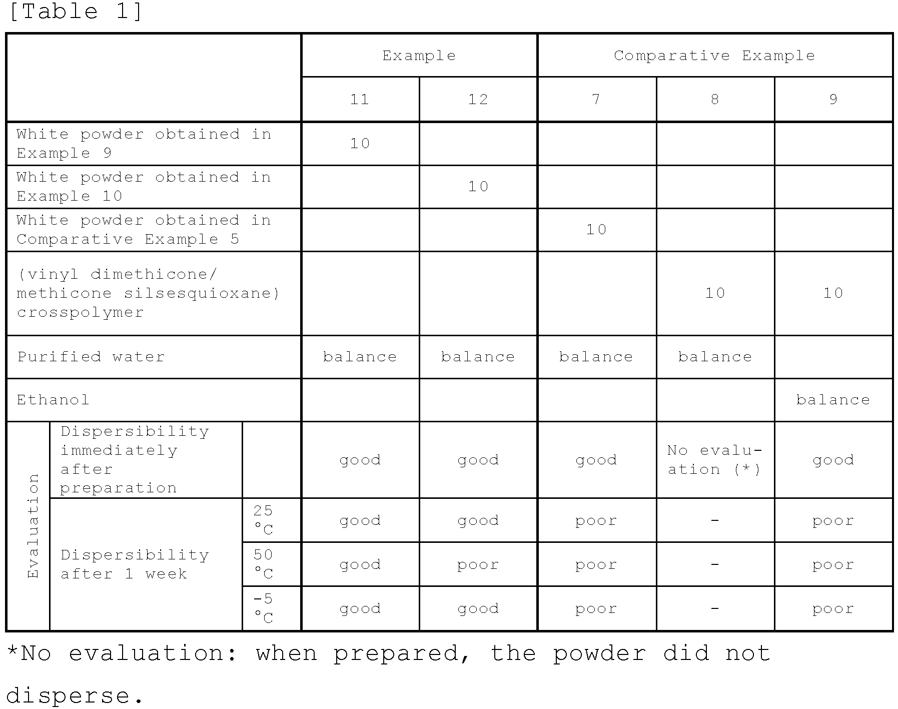

The formulations were adjusted, so that the total of the components was 100.

As shown in Table 1, it was verified that Examples 11, 12 using the dispersible powders are excellent in dispersibility and high in dispersion stability. In Comparative Example 8, the preparation itself was impossible. In Comparative Example 9, the powder dispersed immediately after the preparation, but the precipitation and agglomeration occurred over time, then making it difficult to disperse. Moreover, when only the nonionic surfactant is used to perform the surface treatment as in Comparative Example 7, the dispersibility is not improved. These results revealed that the surface treatment with a (meth)acrylic (co)polymer containing a repeating unit having a phosphorylcholine group as in the present invention results in higher dispersion stability.

### <Examples 13 to 18, Comparative Examples 10 to 16>

Lotions shown in Table 2 were produced according to a conventional method to evaluate the usability.

### [Sensory Evaluation Method]

Approximately 5 g of each lotion obtained according to the formulations shown in Table 2 below was applied to the skin, and subjected to sensory evaluations for spreadability upon application, uniformity, and stretchability. Note that the spreadability upon application refers to touch or application comfortability such as easiness (lightness) and smoothness when applied and spread; and the stretchability refers to a length or area of the cosmetic physically spread. Moreover, regarding the dispersion stability, whether or not the powder agglomerated was visually evaluated based on the product appearance one week after the production. The result is shown by the following evaluation criteria according to the number of panelists who stated "effective".

### [Evaluation Criteria]

excellent: 4 to 5 panelists stated effective
good: 3 panelists stated effective
fair: 2 panelists stated effective
poor: 1 or 0 panelists stated effective

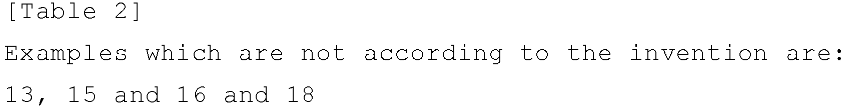

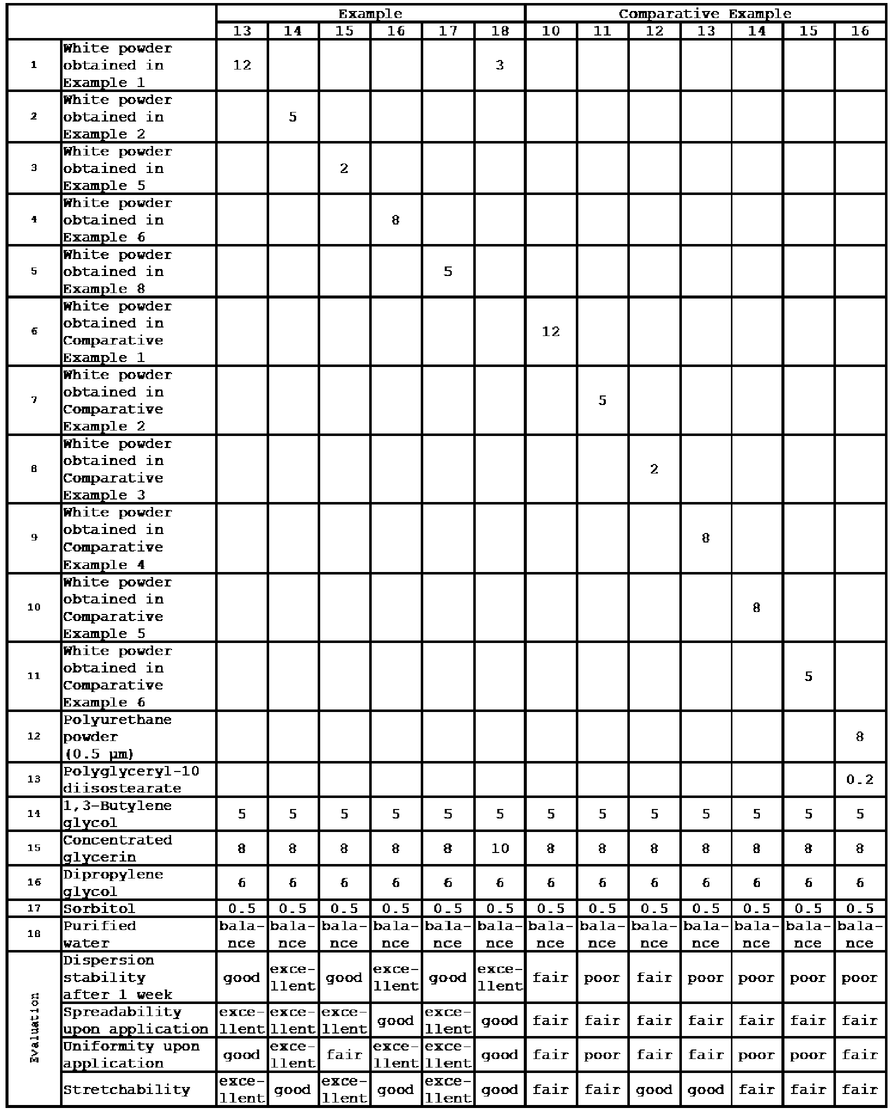

The formulations were adjusted, so that the total of the components was 100.

As shown in Table 2, it was verified that the lotions of Examples 13 to 18 are quite excellent in dispersibility, high in dispersion stability, and form uniform cosmetic films, and the spreadability upon application and stretchability are favorable.

In contrast, in Comparative Examples 10 to 16 using the powders obtained in Comparative Examples 1 to 6, the dispersion stability, spreadability upon application, uniformity, and stretchability were inferior. These results revealed that the inventive dispersible powders are excellent in dispersibility, high in dispersion stability, capable of forming a uniform cosmetic film when blended in a cosmetic, have favorable spreadability upon application and stretchability, and improve the feeling on use.

### [Example 19] O/W Sunscreen (not claimed)

### <Preparation of cosmetic>

A: Components (9) to (15) were homogeneously mixed at 85°C.
B: Components (1) to (6) were homogeneously mixed at 85°C.
C: At 80°C, the mixture B and components (7), (8) were added to the mixture A, emulsified, and gradually cooled. Thus, an O/W sunscreen was obtained.

| | Components | mass(%) |
|---|---|---|
| (1) | Polyglyceryl-10 laurate | 3.5 |
| (2) | Glyceryl stearate (SE) | 3.0 |
| (3) | Behenyl alcohol | 3.0 |
| (4) | Ethylhexyl methoxycinnamate | 5.0 |
| (5) | Isononyl isononanoate | 5.0 |
| (6) | Coco-caprylate/caprate | 5.0 |
| (7) | Silicone-treated fine-particle zinc oxide dispersion (Note 1) | 10.0 |
| (8) | Metallic-soap-treated fine-particle titanium oxide dispersion (Note 2) | 10.0 |
| (9) | Hydroxyethylcellulose | 0.2 |
| (10) | 1,3-Butylene glycol | 10.0 |
| (11) | Ethanol | 6.0 |
| (12) | Ethylhexylglycerin | 0.2 |
| (13) | Bisabolol | 0.2 |
| (14) | White powder of Example 3 | 4.0 |
| (15) | Purified water | balance |
| | Total | 100.0% |

| | | |
|---|---|---|
| (Note 1) manufactured by Shin-Etsu Chemical Co., Ltd.; SPD-Z5 (Note 2) manufactured by Shin-Etsu Chemical Co., Ltd.; SPD-T5 | | |

It was verified that the obtained O/W sunscreen is excellent in feeling on use, applicability, and stability over time.

### [Example 20] Non-Aqueous Sunscreen (not claimed)

### <Preparation of cosmetic>

A: Components (1) to (5) were homogeneously mixed and dissolved.
B: Components (6) to (9) were homogeneously mixed.
C: The mixtures A and B were homogeneously mixed. Thus, a non-aqueous sunscreen was obtained.

| | Components | mass (%) |
|---|---|---|
| (1) | Diisopropyl sebacate | balance |
| (2) | Ethylhexyl methoxycinnamate | 7.5 |
| (3) | Bis-ethylhexyloxyphenol methoxyphenyl triazine | 1.0 |
| (4) | Stearyl glycyrrhetinate | 0.2 |
| (5) | BHT | 0.1 |
| (6) | Silicone/alkyl-modified, partially crosslinked polyglycerin-modified silicone composition (Note 1) | 20.0 |
| (7) | Phenyl-modified partially crosslinked dimethylpolysiloxane composition (Note 2) | 30.0 |
| (8) | Metallic-soap-treated fine-particle titanium oxide dispersion (Note 3) | 20.0 |
| (9) | White powder of Example 4 | 4.0 |
| | Total | 100.0% |

| | | |
|---|---|---|
| (Note 1) manufactured by Shin-Etsu Chemical Co., Ltd.; KSG-850Z (Note 2) manufactured by Shin-Etsu Chemical Co., Ltd.; KSG-18A (Note 3) manufactured by Shin-Etsu Chemical Co., Ltd.; SPD-T5 | | |

It was verified that the obtained non-aqueous sunscreen is excellent in feeling on use, applicability, and stability over time.

### [Example 21] Powder Foundation (not claimed)

### <Preparation of cosmetic>

A: Components 1 to 3 were homogeneously mixed.
B: Components 4 to 12 were homogeneously mixed.
C: The mixture A was added to the mixture B, and homogeneously mixed using a Henschel mixer. The obtained powder was passed through a 100-mesh net, followed by molding in a metal dish using a mold. Thus, a powder foundation was obtained.

| | Components | mass(%) |
|---|---|---|
| 1. | 2-Ethylhexyl para-methoxycinnamate | 4.0 |
| 2. | Diphenylsiloxy phenyl trimethicone (Note 1) | 4.5 |
| 3. | Glyceryl triethylhexanoate | 1.5 |
| 4. | Barium sulfate | 10.0 |
| 5. | White powder of Example 7 | 5.0 |
| 6. | Crosslinked dimethylpolysiloxane (Note 2) | 4.0 |
| 7. | Alkyl/silicone-branched silicone-treated mica (Note 3) | 30.0 |
| 8. | Alkyl/silicone-branched siliconetreated talc (Note 3) | 33.3 |
| 9. | Alkyl/silicone-branched siliconetreated titanium oxide (Note 4) | 6.0 |
| 10. | Alkyl/silicone-branched siliconetreated yellow iron oxide (Note 4) | 1.0 |
| 11. | Alkyl/silicone-branched siliconetreated red iron oxide (Note 4) | 0.5 |
| 12. | Alkyl/silicone-branched siliconetreated black iron oxide (Note 4) | 0.2 |
| | Total | 100.0% |

| | | |
|---|---|---|
| (Note 1) manufactured by Shin-Etsu Chemical Co., Ltd.; KF-56A (Note 2) manufactured by Shin-Etsu Chemical Co., Ltd.; KSG-15 (Note 3) manufactured by Shin-Etsu Chemical Co., Ltd.; treated with KF-9909 (Note 4) manufactured by Shin-Etsu Chemical Co., Ltd.; KTP-09-Series | | |

The obtained powder foundation was fine and spread lightly. The foundation was excellent in adhesion.

### [Example 22] O/W Cream

### <Preparation of cosmetic>

A: Components (4) to (11) were mixed.
B: Components (1) to (3) were mixed, added to the mixture A, and emulsified by stirring.

| | Components | mass(%) |
|---|---|---|
| (1) | Crosslinked dimethylpolysiloxane (Note 1) | 8.0 |
| (2) | Glyceryl triethylhexanoate | 5.0 |
| (3) | Alkyl/silicone-branched siliconetreated titanium oxide (Note 2) | 1.0 |
| (4) | White powder of Example 2 | 2.0 |
| (5) | Dipropylene glycol | 7.0 |
| (6) | Glycerin | 5.0 |
| (7) | Methylcellulose (2% aqueous solution) (Note 3) | 7.0 |
| (8) | Polyacryl amide-based emulsifier (Note 4) | 2.0 |
| (9) | Preservative | 0.1 |
| (10) | Fragrance | 0.2 |
| (11) | Purified water | balance |
| | Total | 100.0% |

| | | |
|---|---|---|
| (Note 1) manufactured by Shin-Etsu Chemical Co., Ltd.; KSG-16 (Note 2) manufactured by Shin-Etsu Chemical Co., Ltd.; KTP-09W (Note 3) manufactured by Shin-Etsu Chemical Co., Ltd.; METOLOSE SM-4000 (Note 4) manufactured by SEPPIC S.A.; SEPIGEL 305 | | |

The obtained O/W cream was spread smoothly without sticking, and had fresh feeling on use and also excellent stability over time.

### [Example 23] Non-Aqueous Concealer (not claimed)

### <Preparation of cosmetic>

A: Components (7) to (12) were prepared into a paste with a three-roll mill.
B: The mixture A and Components (1) to (6) were homogeneously mixed. Thus, a non-aqueous concealer was obtained.

| | Components | mass(%) |
|---|---|---|
| (1) | Phenyl-modified silicone composite powder (Note 1) | 20.00 |
| (2) | White powder of Example 6 | 8.00 |
| (3) | Crosslinked dimethylpolysiloxane (Note 2) | 6.00 |
| (4) | Dimethylpolysiloxane (6cs) | 40.00 |
| (5) | Methylphenylpolysiloxane (Note 3) | 5.00 |
| (6) | Decamethylcyclopentasiloxane | 12.90 |
| (7) | Glyceryl triethylhexanoate | 2.20 |
| (8) | Silicone-branched polyether-modified silicone (Note 4) | 0.50 |
| (9) | Alkyl/silicone-branched silicone- | 5.00 |
| | treated titanium oxide (Note 5) | |
| (10) | Alkyl/silicone-branched silicone-treated yellow iron oxide (Note 5) | 0.25 |
| (11) | Alkyl/silicone-branched silicone-treated red iron oxide (Note 5) | 0.10 |
| (12) | Alkyl/silicone-branched silicone-treated black iron oxide (Note 5) | 0.05 |
| | Total | 100.00% |

| | | |
|---|---|---|
| (Note 1) manufactured by Shin-Etsu Chemical Co., Ltd.; KSP-300 (Note 2) manufactured by Shin-Etsu Chemical Co., Ltd.; KSG-16 (Note 3) manufactured by Shin-Etsu Chemical Co., Ltd.; KF-56A (Note 4) manufactured by Shin-Etsu Chemical Co., Ltd.; KF-6028P (Note 5) manufactured by Shin-Etsu Chemical Co., Ltd.; KTP-09-Series | | |

The spreadability and adhesion of the powder in the obtained non-aqueous concealer were favorable. The non-aqueous concealer had light touch and favorable stability over time.

### [Example 24] Milky Lotion (not claimed)

### <Preparation of cosmetic>

Components (1) to (9) were homogeneously mixed.

| | Components | mass (%) |
|---|---|---|
| (1) | Ethanol | 5.00 |
| (2) | Glycerin | 4.00 |
| (3) | 1,3-Butylene glycol | 3.00 |
| (4) | Purified water | balance |
| (5) | Carboxy vinyl polymer (1% aqueous solution) | 15.00 |
| (6) | Sodium hydroxide | 0.05 |
| (7) | Xanthan gum (2% aqueous solution) | 10.00 |
| (8) | Disodium edetate | 0.05 |
| (9) | White powder of Example 1 | 10.00 |
| Total | 100.00% | |

The obtained milky lotion was spread smoothly, and had favorable smoothness, fresh feeling on use, and excellent stability over time, too.

### [Example 25] Hair Rinse (not claimed)

### <Preparation of cosmetic>

A: Components (1) to (6) were heated and mixed, and further component (10) was added, mixed, and cooled. B: Components (7) to (9) were mixed, added to the mixture A, and thoroughly mixed.

| | Components | mass(%) |
|---|---|---|
| (1) | Cetostearyl alcohol | 2.0 |
| (2) | Cetyl 2-ethylhexanoate | 3.0 |
| (3) | Behentrimonium chloride | 1.0 |
| (4) | Paraben (preservative ) | q.s. |
| (5) | Propylene glycol | 5.0 |
| (6) | Methylphenylpolysiloxane (Note 1) | 1.0 |
| (7) | Hydroxyethylcellulose (1% aqueous solution) | 10.0 |
| (8) | White powder of Example 6 | 5.0 |
| (9) | Fragrance | q.s. |
| (10) | Purified water | balance |
| | Total | 100.0% |

| | | |
|---|---|---|
| (Note 1) manufactured by Shin-Etsu Chemical Co., Ltd.; KF-56A | | |

The obtained hair rinse did not cause the hair to be tangled when rinsed off. The rinsed hair had smooth finish.

### [Example 26] O/W Cosmetic Foundation (not claimed)

### <Preparation of cosmetic>

Components (1) to (8) were homogeneously mixed.

| Components | | mass (%) |
|---|---|---|
| (1) | White powder of Example 3 | 5.00 |
| (2) | Ethanol | 5.00 |
| (3) | 1,3-Butylene glycol | 6.00 |
| (4) | Carboxy vinyl polymer (2% aqueous solution) (Note 1) | 20.00 |
| (5) | Xanthan gum (2% aqueous solution) | 12.00 |
| (6) | Sodium chloride | 0.01 |
| (7) | Preservative | q. s. |
| (8) | Purified water | balance |
| | Total | 100.0% |

| | | |
|---|---|---|
| (Note 1) manufactured by Clariant; ARISTOFLEX AVC | | |

The obtained O/W cosmetic foundation was spread well, and had a beautifying effect by making indistinct the unevenness of the skin. The skin coated therewith had favorable smoothness. Moreover, the stability over time was also favorable.

### [Example 27] Lip Cream

### <Preparation of cosmetic>

A: A part of component (2) was mixed with component (9), and dispersed using a roller mill. Then, the obtained dispersion was mixed with components (1) to (5) while being heated.
B: Components (6) to (8) were heated, added to the mixture obtained in "A", emulsified, and then cooled.
C: Component (10) was added to the emulsified product obtained in "B". Thereby, a lip cream was obtained.

| | Components | mass(%) |
|---|---|---|
| (1) | Dextrin palmitate/ethylhexanoate (Note | 9.0 |
| (2) | Glyceryl triethylhexanoate | 5.0 |
| (3) | Silicone acrylate (Note 2) | 5.0 |
| (4) | Alkyl-modified branched-polyglycerin-modified silicone (Note 3) | 2.0 |
| (5) | Decamethylcyclopentasiloxane | 45.0 |
| (6) | 1,3-Butylene glycol | 5.0 |
| (7) | White powder of Example 8 | 3.0 |
| (8) | Purified water | 16.0 |
| (9) | Iron oxide | q.s. |
| (10) | Mica | q.s. |
| | Total | 100.0% |

| | | |
|---|---|---|
| (Note 1) manufactured by Chiba Flour Milling Co., Ltd.; Rheopearl TT (Note 2) manufactured by Shin-Etsu Chemical Co., Ltd.; KP-561P (Note 3) manufactured by Shin-Etsu Chemical Co., Ltd.; KF-6105 | | |

The obtained lip cream had light spreadability and was free from stickiness and oily feeling, and a highly durable film was formed on the lip. Moreover, the lip cream did not cause bitter or unpleasant sensation when applied, and was easy to use.

## Claims

1. A cosmetic comprising a dispersible powder and water, wherein the dispersible powder is a dispersible powder comprising a powder treated with a surface treatment agent, wherein
the surface treatment agent comprises a (meth)acrylic polymer containing a repeating unit having a phosphorylcholine group, and
a nonionic surfactant having an HLB of 8 to 18, wherein the powder is at least one silicone powder selected from (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, polyalkylsilsesquioxane, and polyarylsilsesquioxane.

2. The cosmetic according to claim 1, wherein the nonionic surfactant is selected from sorbitan fatty acid ester, glycerin fatty acid ester, polyglycerin fatty acid ester, propylene glycol fatty acid ester, polyethylene glycol fatty acid ester, sucrose fatty acid ester, methyl glucoside fatty acid ester, alkyl polyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethylene sorbitol fatty acid ester, polyoxyethylene glycerin fatty acid ester, polyoxyethylene propylene glycol fatty acid ester, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesterol ether, polyether-modified organopolysiloxane, and polyglycerin-modified organopolysiloxane.

3. The cosmetic according to claim 2, wherein the nonionic surfactant is polyglycerin fatty acid ester.

4. The cosmetic according to claim 3, wherein the polyglycerin fatty acid ester is polyglyceryl isostearate.

5. The cosmetic according to claim 1, further comprising a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol.

## Patentansprüche

1. Kosmetikum, das ein dispergierbares Pulver und Wasser umfasst, wobei das dispergierbare Pulver ein dispergierbares Pulver ist, das ein mit einem Oberflächenbehandlungsmittel behandeltes Pulver umfasst, wobei
das Oberflächenbehandlungsmittel ein (Meth)acrylpolymer, das eine Wiederholungseinheit mit einer Phosphorylcholingruppe enthält, und
ein nichtionisches Tensid mit einem HLB-Wert von 8 bis 18 umfasst, wobei das Pulver mindestens ein Silikonpulver, ausgewählt aus (Vinyldimethicon/Methiconsilsesquioxan)-Kreuzpolymer, (Diphenyldimethicon/ Vinyldiphenyldimethicon/Silsesquioxan)-Kreuzpolymer, Polysilikon-1-Kreuzpolymer, Polysilikon-22, Polyalkylsilsesquioxan und Polyarylsilsesquioxan ist.

2. Kosmetikum nach Anspruch 1, wobei das nichtionische Tensid ausgewählt ist aus Sorbitanfettsäureestern, Glycerinfettsäureestern, Polyglycerinfettsäureestern, Propylenglycolfettsäureestern, Polyethylenglycolfettsäureestern, Saccharosefettsäureestern, Methylglucosidfettsäureestern, Alkylpolyglucosiden, Polyoxyethylenalkylethern, Polyoxypropylenalkylethern, Polyoxyethylenalkylphenylethern, Polyoxyethylenfettsäureestern, Polyoxyethylensorbitanfettsäureestern, Polyoxyethylensorbitolfettsäureestern, Polyoxyethylenglycerinfettsäureestern, Polyoxyethylenpropylenglycolfettsäureestern, Polyoxyethylenrizinusöl, Polyoxyethylenhydriertem Rizinusöl, Polyoxyethylenphytostanolethern, Polyoxyethylenphytosterolethern, Polyoxyethylencholestanolethern, Polyoxyethylencholesterolethern, polyethermodifizierten Organopolysiloxanen und polyglycerinmodifizierten Organopolysiloxanen.

3. Kosmetikum nach Anspruch 2, wobei das nichtionische Tensid ein Polyglycerinfettsäureester ist.

4. Kosmetikum nach Anspruch 3, wobei der Polyglycerinfettsäureester Polyglycerylisostearat ist.

5. Kosmetikum nach Anspruch 1, das ferner eine hydrophile Substanz, ausgewählt aus Propylenglykol, Trimethylenglykol, Dipropylenglykol, 1,3-Butylenglykol, Methylpropandiol, Pentylenglykol, Glycerin, Diglycerin, Ethylhexylglycerin, Triglycerin, Polyglycerin und Ethanol umfasst.

## Revendications

1. Produit cosmétique comprenant une poudre dispersible et de l'eau, dans lequel la poudre dispersible est une poudre dispersible comprenant une poudre traitée avec un agent de traitement de surface, dans lequel
l'agent de traitement de surface comprend un polymère (méth)acrylique contenant un motif répétitif comportant un groupe phosphorylcholine, et
un tensioactif non ionique présentant un HLB compris entre 8 et 18, la poudre étant constituée d'au moins une poudre de silicone choisie parmi le polymère réticulé (vinyl diméthicone/méthicone silsesquioxane), le polymère réticulé (diphényl diméthicone/vinyl diphényl diméthicone/silsesquioxane), le polymère croisé de polysilicone-1, le polysilicone-22, le polyalkylsilsesquioxane et le polyarylsilsesquioxane.

2. Produit cosmétique selon la revendication 1, dans lequel l'agent tensioactif non ionique est choisi parmi l'ester d'acides gras de sorbitane, l'ester d'acides gras de glycérine, l'ester d'acides gras de polyglycérine, l'ester d'acides gras de propylène glycol, l'ester d'acides gras de polyéthylène glycol, l'ester d'acides gras de saccharose, l'ester d'acides gras de méthylglucoside, l'alkylpolyglucoside, l'éther alkylé de polyoxyéthylène, l'éther alkylé de polyoxypropylène, l'alkyl phénol polyoxyéthylène éther, d'ester d'acide gras de polyoxyéthylène, d'ester d'acide gras de sorbitane de polyoxyéthylène, d'ester d'acide gras de sorbitol de polyoxyéthylène, d'ester d'acide gras de glycérine de polyoxyéthylène, d'ester d'acide gras de propylène glycol de polyoxyéthylène, l'huile de ricin de polyoxyéthylène, l'huile de ricin hydrogénée de polyoxyéthylène, l'éther de phytostanol de polyoxyéthylène, l'éther de phytostérol de polyoxyéthylène, l'éther de cholestanol polyoxyéthyléné, éther de cholestérol polyoxyéthyléné, organopolysiloxane modifié par un polyéther, et organopolysiloxane modifié par de la polyglycérine.

3. Produit cosmétique selon la revendication 2, dans lequel l'agent tensioactif non ionique est un ester d'acide gras de polyglycérine.

4. Produit cosmétique selon la revendication 3, dans lequel l'ester d'acide gras de polyglycérine est l'isostéarate de polyglycéryle.

5. Produit cosmétique selon la revendication 1, comprenant en outre une substance hydrophile choisie parmi le propylène glycol, le triméthylène glycol, le dipropylène glycol, le 1,3-butylène glycol, le méthylpropanediol, le pentylène glycol, la glycérine, la diglycérine, l'éthylhexylglycérine, la triglycérine, la polyglycérine et l'éthanol.
